(19) Europäisches Patentamt
European Patent Office
Office européen des brevets

(11) **EP 3 050 996 A1**

(12) **EUROPEAN PATENT APPLICATION**
published in accordance with Art. 153(4) EPC

(43) Date of publication:
**03.08.2016 Bulletin 2016/31**

(51) Int Cl.:
*C22F 1/06* (2006.01)  *A61F 2/28* (2006.01)
*A61L 27/00* (2006.01)  *B21C 23/00* (2006.01)
*B21C 23/08* (2006.01)  *B21J 5/00* (2006.01)
*B21K 1/76* (2006.01)  *C23G 1/12* (2006.01)
*C22C 23/06* (2006.01)  *C22F 1/00* (2006.01)

(21) Application number: **13894102.6**

(22) Date of filing: **24.09.2013**

(86) International application number:
**PCT/JP2013/075635**

(87) International publication number:
**WO 2015/044997 (02.04.2015 Gazette 2015/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Olympus Corporation**
**Hachioji-shi**
**Tokyo 192-8507 (JP)**

(72) Inventors:
• **TAMAI, Masato**
  **Tokyo 192-8507, (JP)**
• **SAKAMOTO, Takamitsu**
  **Tokyo 192-8507, (JP)**

• **YAMANAKA, Shigeru**
  **Daitou-shi**
  **Osaka 574-0015 (JP)**
• **HIBI, Genki**
  **Daitou-shi**
  **Osaka 574-0015 (JP)**
• **CYATANI, Masahiro**
  **Daitou-shi**
  **Osaka 574-0015 (JP)**

(74) Representative: **Schicker, Silvia**
**Wuesthoff & Wuesthoff**
**Patentanwälte PartG mbB**
**Schweigerstraße 2**
**81541 München (DE)**

(54) **IMPLANT AND MANUFACTURING METHOD THEREFOR**

(57)    Degradation rate is suppressed to be low by suppressing the occurrence of structural defects. Provided is an implant manufacturing method including a molding step (S1) of molding a molded item by treating a raw-material piece constituted of a biodegradable metal material with plastic processing and a grain-size adjusting step (S2) of increasing metal grain size by applying a heat treatment to the molded item molded in the molding step (S1).

FIG. 8

EP 3 050 996 A1

## Description

{Technical Field}

The present invention relates to an implant and a method of manufacturing the same, and relates, in particular, to a magnesium-alloy implant.

{Background Art}

Magnesium and magnesium alloys have lower weights and higher strengths as compared with other metals, and they are beginning to be put to practical use in portable electronic equipment, automobile parts, and so forth. In addition, because magnesium is characterized by biodegradability, there have been advances in research for applications in absorbable stents and absorbable bone-joining materials (for example, see Patent Literature 1).

In the related art, there is a known method of processing a magnesium-alloy material, in which the plastic processability is enhanced by plastically processing an extruded magnesium-alloy material by exerting a load in a direction parallel to the extrusion direction (for example, see Patent Literature 2).

{Citation List}

{Patent Literature}

{PTL 1} Japanese Unexamined Patent Application, Publication No. 2009-178293
{PTL 2} Publication of Japanese Patent No. 4150219 {Summary of Invention}

{Technical Problem}

However, because a-axes of magnesium-alloy crystals are arranged in the extrusion direction of the magnesium alloy, when a compressing force is exerted in the direction parallel thereto, plastic processing can be performed with less force, and thus, the plastic processability is enhanced. On the other hand, the compressing force acts in the direction orthogonal to c-axes of the metal crystals. In general, when a compressing force acts in the direction orthogonal to the c-axes, there is a problem in that structural defects occur in the crystal structure. When using a magnesium alloy as a biodegradable material in particular, the strength thereof against a load in the thickness direction is decreased, which may cause material damage.

In addition, when employing a biodegradable metal material as a material for manufacturing an implant, there is a problem in that stamping causes metal grains to have finer structures, which increases the number of grain boundaries, thus increasing the degradation rate.

The present invention has been conceived in light of the above-described circumstances, and an object thereof is to provide an implant in which the occurrence of a structural defect is suppressed and that has a low degradation rate and a method of manufacturing the same.

{Solution to Problem}

In order to achieve the above-described object, the present invention provides the following solutions.

A first aspect of the present invention is an implant manufacturing method including a molding step of molding a molded item by treating a raw-material piece constituted of a biodegradable metal material with plastic processing; and a grain-size adjusting step of increasing a metal grain size by applying a heat treatment to the molded item molded in the molding step.

With the first aspect, when the raw-material piece constituted of the biodegradable metal material is treated in the molding step with plastic processing, for example, stamping, the metal grain sizes constituting the biodegradable metal material become finer. The metal grain sizes are increased by subsequently applying the heat treatment to the molded item in the grain-size adjusting step, and thus, it is possible to decrease the degradation rate when the implant is embedded in a living organism as an implant. By doing so, it is possible to manufacture an implant that is degraded after functioning for a long time as a structure after being implanted.

In the above-described first aspect, the molding step may include an extruding step of obtaining a plastically-deformed molded magnesium-alloy material by extruding a magnesium alloy; a cutting step of cutting the molded magnesium-alloy material obtained in the extruding step at an angle of 70° to 110° with respect to the extrusion direction;

and a compressing step of exerting a compressing force on a lump of magnesium-alloy material obtained in the cutting step in a direction orthogonal to the extrusion direction.

By doing so, the plastically-deformed molded magnesium-alloy material in which the c-axes of the magnesium metal crystals are oriented in substantially 90° directions with respect to the extruding direction is obtained by extruding the magnesium alloy in the extruding step. Then, the lump of magnesium-alloy material is obtained in the cutting step by cutting the molded magnesium-alloy material at an angle from 70° to 110° with respect to extrusion direction. Subsequently, the plastically-processed item is manufactured in the compressing step by exerting the compressing force on the lump of magnesium-alloy material in the direction orthogonal to the extrusion direction.

In this case, because the compressing force is exerted mainly in the c-axis direction, the compressing force acts in the direction orthogonal to the slip planes of the metal crystals of the magnesium alloy. By doing so, although the processability is decreased as compared with the case in which the compressing force is exerted in the direction orthogonal to the c-axis, it is possible to manufacture a plastically-processed item having a smaller number of structural defects. In other words, it is possible to manufacture a high-strength plastically-processed item.

The above-described first aspect may include, before the compressing step or in the compressing step, a heating step of heating the lump of magnesium-alloy material.

By doing so, it is possible to enhance the processability by causing non-basal slip to occur.

In the above-described first aspect, in the heating step, the lump of magnesium-alloy material may be heated at a temperature that is greater than 300 °C, or, more preferably, that is equal to or greater 350 °C, and that is equal to or less than the melting point of the magnesium alloy.

By doing so, it is possible to plastically process an alloy containing a rare earth, which is typical in the case of a medical magnesium alloy, more specifically, WE43, without generating cracks.

In the above-described first aspect, in the compressing step, the lump of magnesium-alloy material may be compressed at a reduction ratio equal to or greater than 45 %.

By doing so, it is possible to make the material grain sizes of the magnesium alloy finer and to make the material grain sizes uniform. Specifically, because the re-crystalized grain sizes reach near equilibrium at a reduction ratio of about 45 % when the compressing force is gradually exerted on the lump of magnesium-alloy material, it is possible to enhance the corrosion resistance by making the material grain sizes uniform. Here, the reduction ratio can be calculated as follows:

```
Reduction ratio =([thickness before compression] - [thickness

after compression])/[thickness before compression] × 100 (%).
```

In the above-described first aspect, in the compressing step, the compressing force may be exerted by using a metal mold in a state in which a lubricant is applied between the lump of magnesium-alloy material and the metal mold.

By doing so, the pressure exerted on the lump of magnesium-alloy material from the metal mold is dispersed by the lubricant, thus making it possible to approach an even deformation.

In the above-described first aspect, it is preferable that, in the compressing step, applying the lubricant between the metal mold and the lump of magnesium-alloy material and exerting the compressing force be repeated at least twice.

By doing so, even if a newly-formed metal surface is created in association with plastic processing, it is possible to protect the newly-formed metal surface with the lubricant so that the newly-formed metal surface does not come into direct contact with the metal mold to cause adhesion or burning. As a result, it is possible to prevent the occurrence of defective products and the occurrence of damage to the metal mold.

The above-described first aspect may include a shearing step of cutting out a product from the compressed magnesium-alloy material after the compressing step, wherein the shearing step is performed at a reduction rate of 1.5 mm/s or less.

By doing so, a plastically-processed product is manufactured by subjecting the magnesium-alloy material compressed in the compressing step to the shearing step. The shearing step involves, for example, punching process by means of a press apparatus. In this case, in association with shearing, a relatively-smooth sheared portion (sheared surface) and a fractured portion (fractured surface) that is instantaneously separated to have a rough surface are created at the cut surface. Here, by performing the shearing step at a reduction rate equal to or less than 1.5 mm/s, the fractured portion at the cut surface is suppressed to 50 % or less. By doing so, it is possible to manufacture a high-strength plastically-processed product by decreasing the fraction of the fractured region, which is a cause of stress concentration.

The above-described first aspect may include a washing step of washing a surface of the molded item molded in the molding step; and a checking step of checking the impurity concentration at the surface of the molded item that has been washed in the washing step, wherein, in the grain-size adjusting step, the heat treatment is applied to the molded item in the case in which the impurity concentration checked in the checking step is equal to or less than a predetermined value.

By doing so, the metal component remains, as an impurity, on the surface of the molded item that has come into contact with the metal mold when treated with plastic processing. The residual impurity is washed in the washing step, and the concentration thereof is subsequently checked in the checking step. Then, because the grain-size adjusting step is performed in the case in which the impurity concentration on the surface of the molded item is equal to or less than the predetermined value, it is possible to manufacture an implant having a more stable degradation rate by preventing the biodegradable metal material and the impurity metal material from reacting with each other during the heat treatment.

In the above-described first aspect, the washing step may involve a treatment for peeling the surface of the molded item.

By doing so, it is possible to reliably remove the impurity metal material attached to the surface of the molded item together with the surface portion of the molded item that has been peeled off.

In the above-described first aspect, the washing step may involve a treatment for dissolving the surface of the molded item by using an acid.

By doing so, the impurity metal material attached to the surface of the molded item is dissolved by the acid and is removed together with the surface portion of the molded item.

In the above-described first aspect, the washing step may include a treatment for dissolving the surface of the molded item by using an acid and a subsequent treatment for immersing the molded item in an alkaline solution.

By doing so, the impurity metal material attached to the surface of the molded item is dissolved by the acid and removed together with the surface portion of the molded item. Then, the dissolution reaction by the acid can be stopped by means of immersion in the alkaline solution.

In the above-described first aspect, the grain-size adjusting step may involve a solutionizing treatment.

By doing so, it is possible to decrease the degradation rate by increasing, by means of the solutionizing treatment, the grain sizes that have become finer due to plastic processing, and thus, it is also possible to enhance the strength of the molded item.

In the above-described first aspect, in the grain-size adjusting step, an aging precipitation treatment may be performed after the solutionizing treatment.

By doing so, it is possible to decrease the degradation rate by increasing, by means of the solutionizing treatment, the grain sizes that have become finer due to plastic processing. In addition, it is possible to further enhance the strength of the molded item by means of the aging precipitation treatment that follows the solutionizing treatment.

A second aspect of the present invention is a magnesium-alloy implant in which c-axes of metal crystals are oriented in a main load direction.

As described above, because a deformation caused by a compressing force parallel to the c-axes is harder to achieve than a deformation caused by a compressing force orthogonal to the c-axes, it is possible to enhance the strength by orienting the c-axes in the main load direction.

In the above-described second aspect, an average value of deviation angles of normals at (0001) planes of metal crystals with respect to a thickness direction may be equal to or less than 25°.

By doing so, it is possible to decrease the number of structural defects and to enhance the strength against a load in the thickness direction by orienting the c-axes of the metal crystals of the magnesium alloy nearly in the thickness direction.

In the above-described second aspect, an average value of deviation angles of normals at (0001) planes of metal crystals with respect to a direction parallel to a surface may be equal to or greater than 80°, and a width of a cumulative distribution of the deviation angles that are from 16 to 84 % of a maximum value of the deviation angles may be equal to or less than 50°.

By doing so also, it is possible to decrease the number of structural defects and to enhance the strength against a load in the thickness direction by orienting the c-axes of the metal crystals of the magnesium alloy nearly in the thickness direction.

The above-described second aspect may be manufactured by punching processing.

In this case, it is preferable that a fraction of sheared portions at a cut surface along a thickness direction formed by punching processing be equal to or greater than 50 % relative to the thickness.

By doing so, it is possible to provide a high-strength magnesium-alloy implant by decreasing the fraction of the fractured portion, which is a cause of stress concentration.

In the above-described second aspect, at a surface thereof, an Fe-ion concentration may be equal to or less than 0.02 %, a Cu-ion concentration may be equal to or less than 0.15 %, and a nickel concentration may be equal to or less than 0.01 %.

{Advantageous Effects of Invention}

The present invention affords an advantage in that it is possible to manufacture an implant having a low degradation rate by preventing the occurrence of a structural defect.

{Brief Description of Drawings}

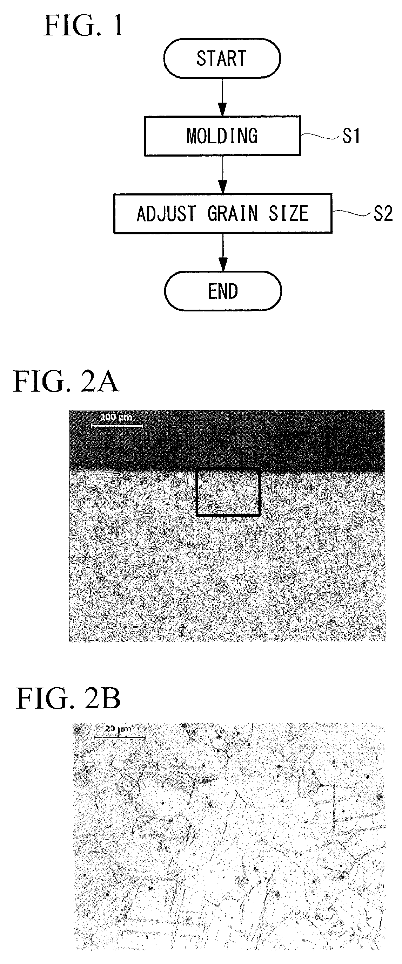

{Fig. 1} Fig. 1 is a flowchart showing an implant manufacturing method according to a first embodiment of the present invention.

{Fig. 2A} Fig. 2A is a photomicrograph showing the crystal structure of a molded item produced in a molding step of the manufacturing method in Fig. 1.

{Fig. 2B} Fig. 2B is a photomicrograph showing, in an enlarged view, the crystal structure of the molded item produced in the molding step of the manufacturing method in Fig. 1.

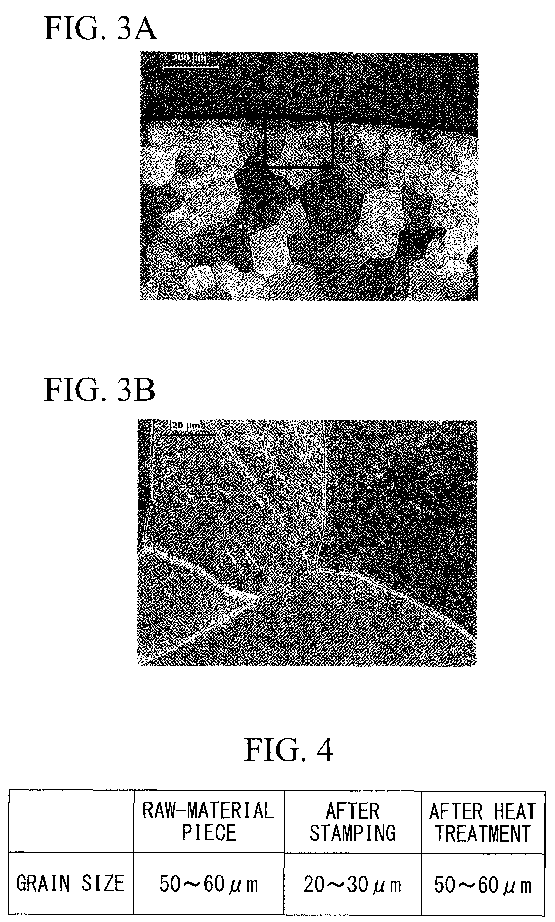

{Fig. 3A} Fig. 3A is a photomicrograph showing the crystal structure of the molded item after a grain-size adjusting step of the manufacturing method in Fig. 1.

{Fig. 3B} Fig. 3B is a photomicrograph showing, in an enlarged view, the crystal structure of the molded item after the grain-size adjusting step of the manufacturing method in Fig. 1.

{Fig. 4} Fig. 4 is a diagram showing a table of changes in grain sizes caused by the manufacturing method in Fig. 1.

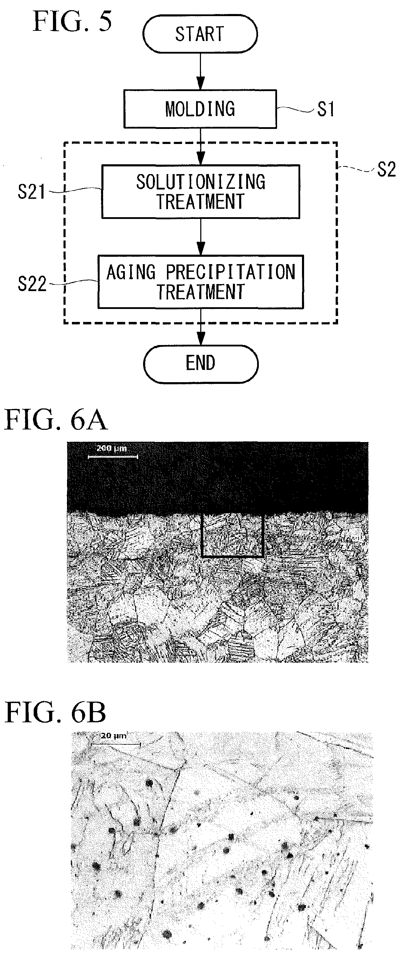

{Fig. 5} Fig. 5 is a flowchart showing a modification of the manufacturing method in Fig. 1.

{Fig. 6A} Fig. 6A is a photomicrograph showing the crystal structure of a molded item produced in a molding step of the manufacturing method in Fig. 5.

{Fig. 6B} Fig. 6B is a photomicrograph showing, in an enlarged view, the crystal structure of the molded item produced in the molding step of the manufacturing method in Fig. 5.

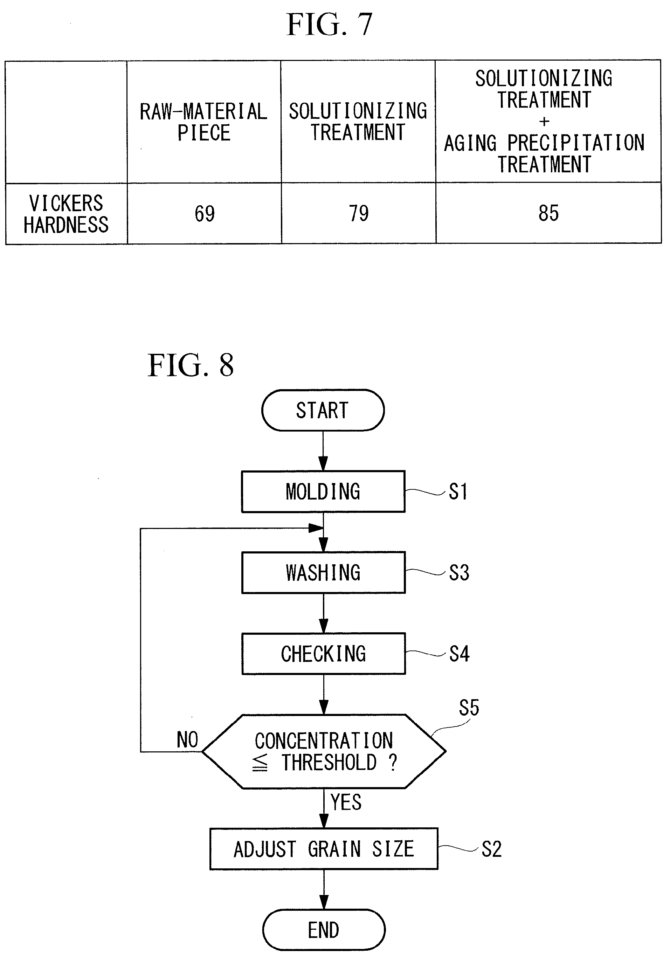

{Fig. 7} Fig. 7 is a diagram showing a table of mechanical strengths of implants based on the manufacturing methods in Fig. 1 and Fig. 5.

{Fig. 8} Fig. 8 is a flowchart showing an implant manufacturing method according to a second embodiment of the present invention.

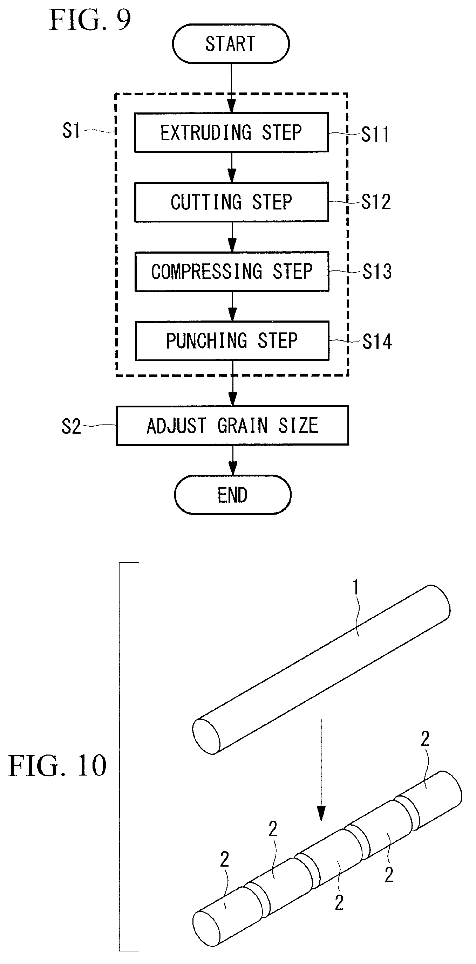

{Fig. 9} Fig. 9 is a flowchart showing an implant manufacturing method according to a third embodiment of the present invention.

{Fig. 10} Fig. 10 is a diagram for explaining a cutting step of a molding step in Fig. 9.

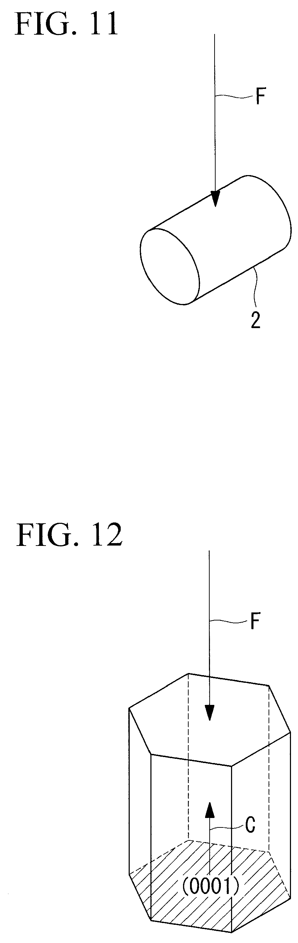

{Fig. 11} Fig. 11 is a diagram for explaining a compressing step of the molding step in Fig. 9.

{Fig. 12} Fig. 12 is a diagram for explaining the direction of the compressing force that acts on metal crystals in the compressing step in Fig. 11.

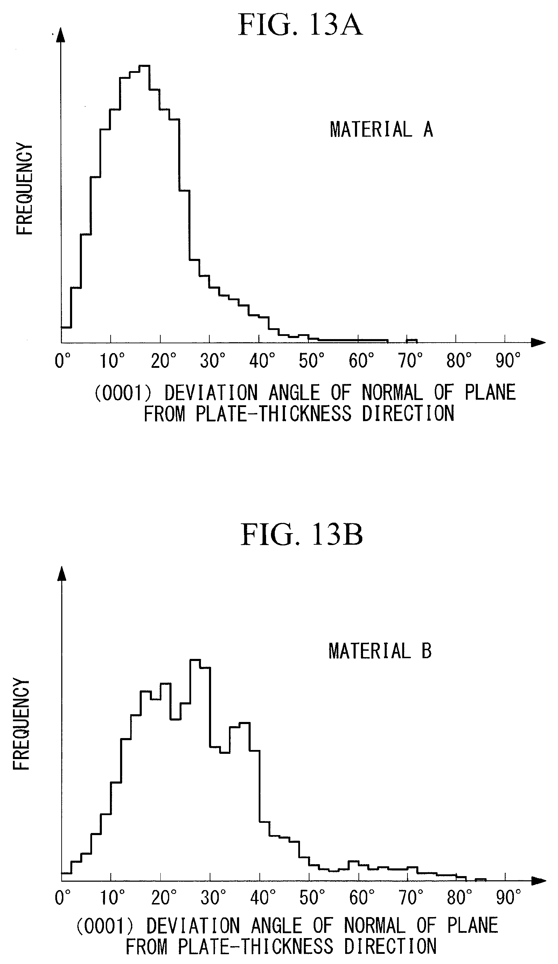

{Fig. 13A} Fig. 13A is a diagram for Example 1 of the molding step of the third embodiment, showing a histogram of the deviation angles of normals at (0001) planes of metal crystals of a material A, which is processed in accordance with the compressing step in Fig. 11, with respect to the plate-thickness direction.

{Fig. 13B} Fig. 13B is a diagram showing, as a comparative example, a histogram of the deviation angles for a material B.

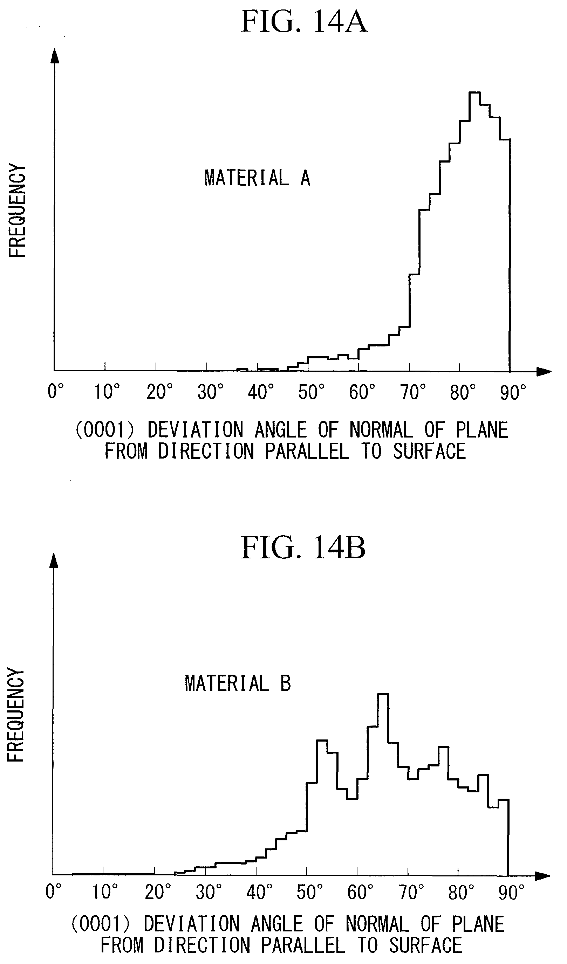

{Fig. 14A} Fig. 14A is a diagram showing a histogram of the deviation angles of the normals at the (0001) planes of the metal crystals of the material A with respect to the direction parallel to a product surface.

{Fig. 14B} Fig. 14B is a diagram showing, as a comparative example, a histogram of the deviation angles for the material B.

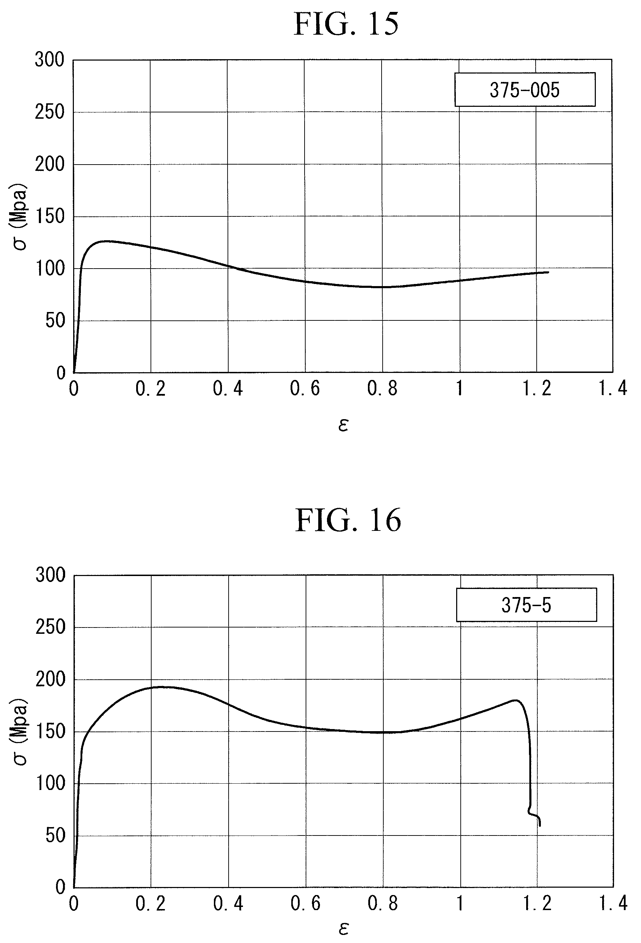

{Fig. 15} Fig. 15 is a graph for Example 2 of the molding step of the third embodiment, showing the relationship between the true strain rate and the load in the case in which the processing conditions for the compressing step in Fig. 11 are such that the heating temperature is 375 °C and the reduction rate is 0.05 mm.

{Fig. 16} Fig. 16 is a graph for Example 2 of the molding step of the third embodiment, showing the relationship between the true strain rate and the load in the case in which the processing conditions for the compressing step in Fig. 11 are such that the heating temperature is 375 °C and the reduction rate is 5 mm.

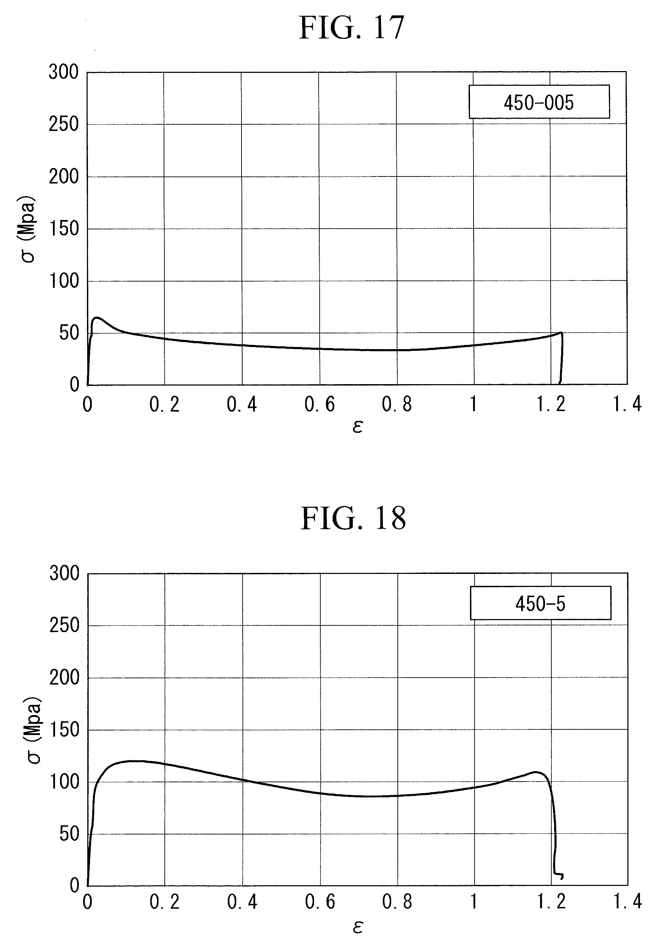

{Fig. 17} Fig. 17 is a graph for Example 2 of the molding step of the third embodiment, showing the relationship between the true strain rate and the load in the case in which the processing conditions for the compressing step in Fig. 11 are such that the heating temperature is 450 °C and the reduction rate is 0.05 mm.

{Fig. 18} Fig. 18 is a graph for Example 2 of the molding step of the third embodiment, showing the relationship between the true strain rate and the load in the case in which the processing conditions for the compressing step in Fig. 11 are such that the heating temperature is 450 °C and the reduction rate is 5 mm.

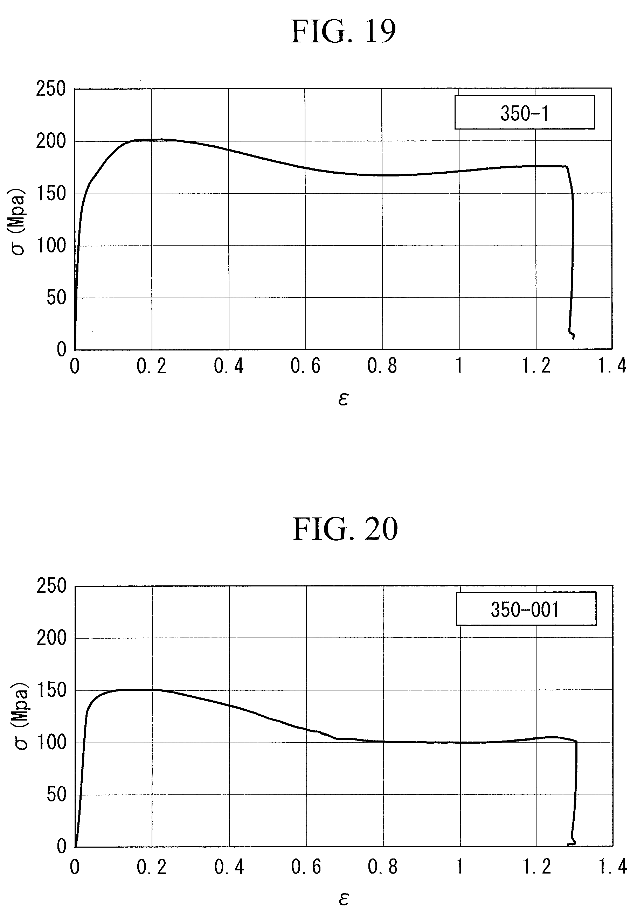

{Fig. 19} Fig. 19 is a graph for Example 2 of the molding step of the third embodiment, showing the relationship between the true strain rate and the load in the case in which the processing conditions for the compressing step in Fig. 11 are such that the heating temperature is 350 °C and the reduction rate is 1 mm.

{Fig. 20} Fig. 20 is a graph for Example 2 of the molding step of the third embodiment, showing the relationship between the true strain rate and the load in the case in which the processing conditions for the compressing step in Fig. 11 are such that the heating temperature is 350 °C and the reduction rate is 0.01 mm.

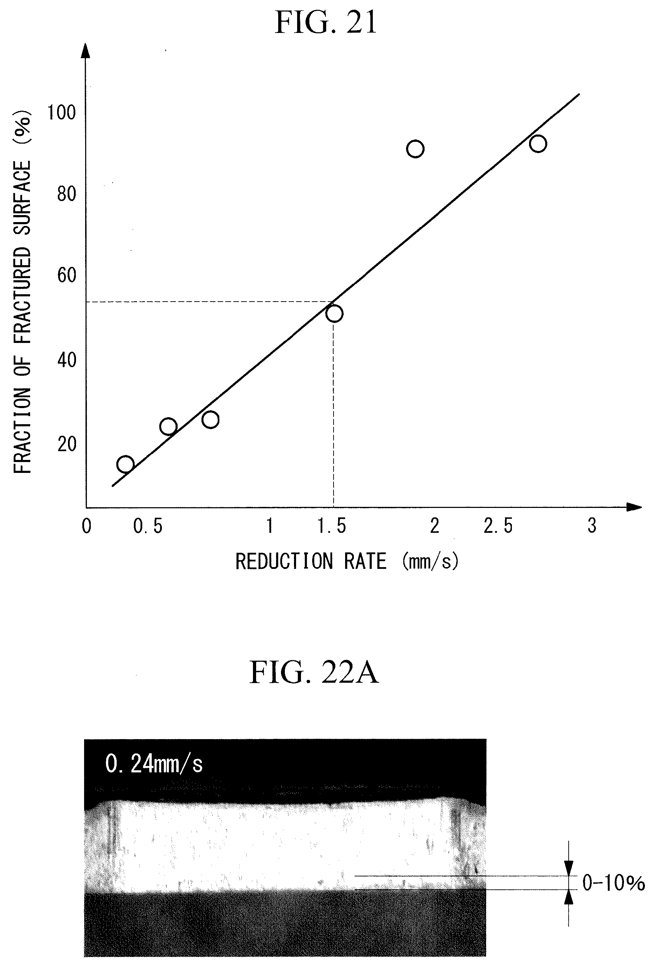

{Fig. 21} Fig. 21 is a diagram showing the relationship between the reduction rate and the fraction of a fractured surface portion in a cut surface formed in the thickness direction in a punching step of the molding step in Fig. 9.

{Fig. 22A} Fig. 22A is a photomicrograph of a cut surface when the reduction rate is 0.24 mm/s.

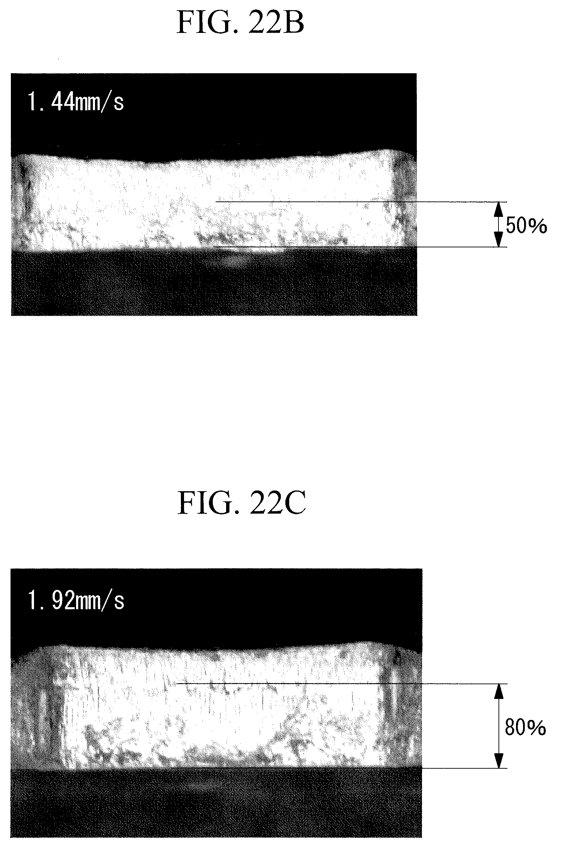

{Fig. 22B} Fig. 22B is a photomicrograph of a cut surface when the reduction rate is 1.44 mm/s.

{Fig. 22C} Fig. 22C is a photomicrograph of a cut surface when the reduction rate is 1.92 mm/s.

{Description of Embodiments}

{First Embodiment}

An implant manufacturing method according to a first embodiment of the present invention will be described below with reference to Figs. 1 to 7.

An implant that is manufactured by using the manufacturing method according to this embodiment is, for example, an implant to be used for joining bones, and is constituted of a biodegradable metal material, for example, a magnesium-alloy material (for example, WE43).

As shown in Fig. 1, the implant manufacturing method according to this embodiment includes a molding step S1 of molding a molded item by treating a raw-material piece constituted of a biodegradable metal material with hot plastic processing, such as stamping, and a grain-size adjusting step S2 of applying heat treatment, which increases the grain size, to the molded item molded in the molding step S1.

The molding step S1 is a step of stamping for, for example, 1 minute at 300 °C and 100 MPa. The temperature, pressure, and processing time for stamping are mere examples, and it is permissible to employ other conditions.

The grain-size adjusting step S2 is a step of air cooling after applying heat treatment (solutionizing treatment) for, for example, eight hours at 525 °C. The temperature and treatment time for the heat treatment are mere examples, and it is permissible to employ other conditions.

With the thus-configured implant manufacturing method according to this embodiment, by being subjected to stamping in the molding step S1, a molded item having a desired shape is manufactured from the raw-material piece. At this time, the grain sizes of the biodegradable metal material become finer, as shown in Figs. 2A and 2B. Fig. 2B is an enlarged photomicrograph of the portion surrounded by a rectangle in Fig. 2A.

Then, by being subjected to the heat treatment in the grain-size adjusting step S2, the grain sizes of the biodegradable metal material constituting the molded item that has been molded are increased, as shown in Figs. 3A and 3B. Fig. 3B is an enlarged photomicrograph of the portion surrounded by a rectangle in Fig. 3A.

In addition, Fig. 4 shows the grain sizes of the biodegradable metal material in the raw-material piece, after stamping, and after the heat treatment, respectively.

With the thus-configured implant manufacturing method according to this embodiment, the grain sizes of the biodegradable metal material that have been made finer by stamping in the molding step S1 are increased by the heat treatment in the grain-size adjusting step S2. By doing so, the number of the grain boundaries at the surface of the molded item are decreased, and thus, it is possible to decrease the degradation rate. Specifically, there is an advantage in that the implant implanted to serve as a structural material for bonding bones can maintain a sufficient strength to serve as the structural material for a long time after being implanted, thus making it possible to assist bone joining by bone formation. Then, after bone joining is completed, the implant is degraded and eliminated over time, thus preventing a foreign object from remaining in the body interior.

Note that, although the solutionizing treatment is performed in the grain-size adjusting step S2 in this embodiment, additionally, heat treatment (aging precipitation treatment (step S22)) may be performed for 6 hours at 250 °C after the solutionizing treatment (step S21), as shown in Fig. 5.

By doing so, as shown in Fig. 7, it is possible to enhance the mechanical strength (Vickers hardness) of the implant, while maintaining large grain sizes, as shown in Figs. 6A and 6B. Fig. 6B is an enlarged photomicrograph of the portion surrounded by a rectangle in Fig. 6A.

{Second Embodiment}

Next, an implant manufacturing method according to a second embodiment of the present invention will be described below with reference to Fig. 8.

In the description of this embodiment, portions having the same configurations as those of the above-described first embodiment are given the same reference signs, and descriptions thereof will be omitted.

As shown in Fig. 8, the manufacturing method according to this embodiment includes, between the molding step S1 and the grain-size adjusting step S2, a washing step S3 of washing the molded item that has been molded, a checking step S4 of checking the impurity concentration at the surface of the molded item, and a judging step S5 of judging whether or not the impurity concentration checked in the checking step S4 is below a predetermined threshold.

The washing step S3 is a step of degreasing the molded item, immersing the molded item in an alkaline solution after immersion in an acidic solution, and subsequently drying the molded item.

The checking step S4 is a step of measuring the concentration of Fe ions that have leached out into the acidic solution.

So long as chromic acid, boric acid, or the like, which affects a living organism, is excluded, phosphoric acid, hydrochloric acid, or the like may be used in the acidic solution.

In the judging step S5, it is judged whether or not the Fe-ion concentration measured in the checking step S4 is equal to or less than the predetermined threshold. Then, in the judging step S5, if the Fe-ion concentration is equal to or less than the threshold, the process advances to the grain-size adjusting step S2, and, if the Fe-ion concentration exceeds the threshold, steps from the washing step S3 are repeated again.

With the thus-configured implant manufacturing method according to this embodiment, by performing stamping by using an iron metal mold, the iron component constituting the metal mold becomes attached to the surface of the molded item. To cope with this, the iron component attached to the surface of the molded item is dissolved by acid by immersing the molded item in the acidic solution in the washing step S3, thus making the iron component leach out into the acidic solution in the form of Fe ions. By doing so, it is possible to remove the iron component, which is an impurity metal material on the surface of the molded item.

If the heat treatment is performed in the state in which the iron component is left attached on the surface, corrosion of the magnesium alloy advances with the iron component serving as a starting point. As a result, the processing precision and the mechanical strength of the heat-treated implant are deteriorated. With this embodiment, because the iron component on the surface of the molded item is removed, there is an advantage in that it is possible to manufacture a high-precision, high-strength implant by suppressing the advancement of corrosion during the heat treatment step.

As for the predetermined threshold, the amount of the iron component that becomes attached to the molded item from the metal mold during stamping may be experimentally determined, and the predetermined threshold may be set based on the result thereof.

In addition, in the checking step S4, the concentration may be measured after immersion in the acidic solution for a predetermined amount of time; alternatively, Fe ions in the acidic solution may be measured over time to determine the rate of change of the concentration thereof, and the Fe-ion concentration at the point in time when the change in the concentration fell below a predetermined threshold may be measured.

In addition, the Fe-ion concentration may be measured by directly analyzing the surface composition of the implant by using an X-ray fluorescence device.

Note that, although implants for joining bones have been described as examples, in the individual embodiments described above, alternatively, the present invention may be applied to manufacturing of other arbitrary implants.

In addition, although the magnesium alloy has been described as an example of a biodegradable metal material, the present invention may be applied to other arbitrary biodegradable materials.

In addition, although the iron component has been described as an example of an impurity metal material that becomes attached to the surface of the molded item, alternatively or in addition thereto, a copper component or a nickel component may be removed by washing.

In addition, in the above-described embodiment, although an impurity metal material is removed by immersing the molded item in the acidic solution in the washing step S3, alternatively, the impurity metal material may be removed by peeling it off from the surface of the molded item by means of cutting or the like.

It is desirable that the Fe-ion concentration remaining on the implant surface be 0.02 % or less, that the Cu-ion concentration be 0.15 % or less, and that the nickel concentration be 0.01 % or less.

{Third Embodiment}

Next, an implant manufacturing method according to a third embodiment of the present invention will be described below with reference to Figs. 9 to 22C.

As shown in Fig. 9, the implant manufacturing method according to this embodiment differs from that of the first embodiment in terms of the molding step S1. Therefore, in this embodiment, the molding step S1 will mainly be described, and descriptions of other steps S2, S21, and S22 that are the same as those of the first embodiment will be omitted. The implant manufacturing method of this embodiment may additionally be provided with the washing step S3, the checking step S4, and the judging step S5 described in the second embodiment.

As shown in Fig. 9, the molding step S1 of the implant manufacturing method according to this embodiment includes an extruding step S11 of obtaining a plastically-deformed magnesium-alloy material by extruding a magnesium-alloy material (raw-material piece), a cutting step S12 of cutting the extruded magnesium-alloy material, and a compressing step S13 of exerting a compressing force on the cut lump of magnesium-alloy material, and a punching step S14 of punching out a product from the magnesium-alloy material compressed in the compressing step.

The extruding step S11 is a step of plastically deforming the magnesium-alloy material into a rod-shaped extruded material 1 having a predetermined lateral cross-sectional shape by using a die. Depending on the extruding conditions, orientations of metal crystals are changed so that (0001) planes of the metal crystals become oriented substantially parallel to the extrusion direction.

As shown in Fig. 10, the cutting step S12 is a step of obtaining lumps of magnesium-alloy material 2 that are divided in the longitudinal direction by cutting the extruded material 1 manufactured in the extruding step S11 at 70° to 110°, that is, in a direction substantially orthogonal to the longitudinal direction thereof.

Actually, it is preferable that the lumps of magnesium-alloy material 2 be obtained by measuring the orientations of the (0001) planes of the extruded material 1 by means of pole figure measurement or the like and by cutting at an angle orthogonal to a surface in which the (0001) planes are mostly oriented.

As shown in Fig. 11, the compressing step S13 is a step of rolling the lump of magnesium-alloy material 2 into a plate by exerting a compressing force F from a reduction metal mold onto the lump of magnesium-alloy material 2 obtained in the cutting step S12 in the direction orthogonal to the extrusion direction in the extruding step S11. By doing so, as shown in Fig. 12, the compressing force F is exerted substantially parallel to c-axes, which are orthogonal to the (0001) planes, of metal crystals of the magnesium alloy constituting the lump of magnesium-alloy material 2.

In the compressing step S13, the compressing force F is exerted in the state in which the lump of magnesium-alloy material 2 is heated. It is preferable that the heating temperature be equal to or greater than 100 °C, which is a temperature at which non-basal slip occurs; in particular, when using WE43, which is a medical magnesium-alloy material, it is preferable that the temperature be greater than 300 °C, and it is more preferable that the temperature be equal to or greater than 350 °C.

In addition, the compressing step S13 is performed by applying a lubricant between the metal mold and the lump of magnesium-alloy material 2. A solid lubricant, a fluid lubricant, or the like can be employed as the lubricant.

Also, the compressing step S13 is performed by repeating, multiple times, the step of applying the lubricant and the step of reducing the lump of magnesium-alloy material 2 by using the metal mold.

Furthermore, the compressing step S13 is configured so as to compress the lump of magnesium-alloy material 2 by a reduction ratio of 45 % or greater.

Here, the reduction ratio is expressed by the expression below.

```
Reduction ratio = ([thickness before compression] - [thickness

after compression])/[thickness before compression] × 100 (%)
```

The punching step S14 is a step of punching out the plate-like magnesium-alloy material obtained in the compressing step S13 by using a punch die. In this punching step S14, it is preferable that the rate at which the magnesium-alloy material is reduced by using the metal mold be suppressed to 1.5 mm/s or less.

The operation of the thus-configured implant manufacturing method according to this embodiment will be described below.

In order to plastically process a magnesium-alloy material by using the implant manufacturing method according to this embodiment, first, the rod-shaped extruded material 1 is obtained by extruding the magnesium-alloy raw material in the extruding step S11.

In this extruding step S11, the orientations of metal crystals of the magnesium-alloy material are changed so that the (0001) planes of the metal crystals are oriented substantially parallel to the extrusion direction. The extruded material 1 obtained in this way is subjected to the cutting step S12. In the cutting step S12, a plurality of lumps of magnesium-alloy material 2 are obtained by cutting the extruded material 1 at cut surfaces orthogonal to the extrusion direction. Specifically, the cut surfaces are arranged in the direction orthogonal to the (0001) planes of the metal crystals.

Next, the individual lumps of magnesium-alloy material 2 are subjected to the compressing step S13. In the compressing step S13, in the state in which the lumps of magnesium-alloy material 2 are heated to 100 °C or greater, the compressing force F is exerted on the lumps of magnesium-alloy material 2 in the direction orthogonal to the extrusion direction. The compressing force F is exerted in a direction substantially parallel to the c-axes of the metal crystals that are oriented in substantially one direction at positions in the lumps of magnesium-alloy material 2 where the diameters thereof makes the degree of processing greatest.

This direction of the compressing force F in the compressing step S13 is the direction orthogonal to slip planes of the metal crystals, and, although the ease of plastic processing is decreased as compared with a case in which the compressing force F is exerted parallel to the slip planes, there is an advantage in that structural defects of the metal crystals are less likely to occur. In other words, there is an advantage in that, because the occurrence of structural defects in the compressed magnesium-alloy material is suppressed, it is possible to enhance the mechanical strength.

In this case, because the compressing force F is exerted on the lumps of magnesium-alloy material 2 in the state in which the lumps of magnesium-alloy material 2 are heated to 100 °C or greater, as compared with the case in which heating is not performed, the processability is enhanced by the occurrence of non-basal slip.

In addition, in the compressing step S13, the compressing force F is exerted in the state in which the lubricant is applied between the metal mold and the lump of magnesium-alloy material 2. By doing so, the compressing force F is dispersed over the entire contact surface between the lump of magnesium-alloy material 2 and the metal mold by the action of the lubricant, and thus, it is possible to evenly exert the compressing force F over the entirety of the lump of magnesium-alloy material 2.

When the compressing force F is exerted in the compressing step S13, the magnesium-alloy material is refined, thus decreasing the grain size thereof. Assuming a medical application, there is a known relationship between the uniformity of material grain sizes and the corrosion resistance of the material, and, when imparting a material with corrosion resistance, it is desirable to make the material grain sizes uniform. In general, when a compression load is exerted on a material, elastic deformation occurs first, and, when the compression load enters the plastic region, plastic deformation occurs while material grains become finer in association therewith. The crystal sizes reach equilibrium when further compression load is exerted, and thus, the material grain sizes become uniform.

In other words, it is desirable to exert the compression load until the material grain sizes become uniform. Specifically, the magnesium-alloy material is compressed so that the reduction ratio becomes equal to or greater than 45 %, thus making the material grains finer to achieve uniform grain sizes. When the compression force F is gradually exerted on the lump of magnesium-alloy material 2, because re-crystalized grain sizes reach near equilibrium at a reduction ratio of about 45 %, it is possible to make the material grain sizes uniform. Thus, particularly in the case in which the manufactured product is a product used for medical usage, such as a magnesium-alloy implant, there is an advantage in that it is possible to enhance the corrosion resistance by achieving uniform grain sizes.

Then, the plate-like magnesium-alloy material obtained in the compressing step S13 is subjected to the punching step S14. The punching step S14 is a step of obtaining the molded item by removing unnecessary portions by means of punching by using a stamping machine in order to set the product shape.

Because punching is performed by means of shearing by pressing the punch die against the plate-like magnesium-alloy material in the plate-thickness direction thereof, although cut surfaces are formed in the thickness direction, the fraction of sheared surface portions at the cut surfaces relative to that of fractured surface portions can be increased by suppressing the reduction rate of the metal mold to 1.5 mm/s or less. As a result, there is an advantage in that it is possible to enhance the strength of a product, a magnesium-alloy implant in particular, by decreasing the factors causing stress concentration by decreasing the fraction of the fractured surface portions.

As has been described above, with the implant manufacturing method according to this embodiment, there is an advantage in that it is possible to manufacture a high-strength plastically-processed item by suppressing the occurrence of structural defects after compression.

Next, examples of the molding step S1 of this embodiment will be described below.

(Example 1)

Example 1 shows the relationship between the direction in which the compressing force F is exerted on the lump of magnesium-alloy material 2 and the orientations of metal crystals at the surface of the compressed plate-like magnesium-alloy material.

Regarding the orientations of metal crystals at the surface of the plate-like magnesium-alloy material, Fig. 13A shows the case in which the compressing force F is exerted in the vertical direction with respect to the extrusion direction in the extruding step S11, as with the manufacturing method according to this embodiment (material A), and Fig. 13B shows the case in which the compressing force F is exerted in the direction parallel to the extrusion direction (material B).

Figs. 13A and 13B are histograms in which the lateral axes show deviation angles, with respect to the plate-thickness direction, of normals at the (0001) planes of the compressed metal crystals and the vertical axes show the frequencies.

Based on Figs. 13A and 13B, average values of the deviation angles and integrated widths for the respective compression methods are calculated and shown in Table 1.

{Table 1}

| MATERIAL | DEVIATION ANGLE FROM PLATE-THICKNESS DIRECTION | INTEGRATED WIDTH OF DEVIATION ANGLE |
|---|---|---|
| MATERIAL A | 16.8° | 17.6° |
| MATERIAL B | 26.0° | 25.2° |

The integrated widths of the deviation angles are widths of the deviation angles that fall within a range between 16 % and 84 % of the maximum value of the deviation angles in the cumulative distribution curves in Figs. 13A and 13B.

As shown in Table 1, the average value of the deviation angle is 16.8° for the material A, whereas the average value of the deviation angle is 26.0° for the material B, thus indicating that a large change occurred in the crystal orientation due to the action of the compressing force F in the material B. Therefore, a product employing a material for which the average value of the deviation angle is equal to or less than 25° is a product in which the compressing force F is exerted in the vertical direction with respect to the extrusion direction, and thus, it is possible to decrease the number of structural defects and to enhance the strength.

In addition, as shown in Table 1, the integrated width of the deviation angle is 17.6° for the material A, whereas the average value of the deviation angle is 25.2° for the material B, and thus, on the basis of this also, it is shown that a large change occurred in the crystal orientation due to the action of the compressing force F in the material B. Therefore, a product employing a material for which the integrated width of the deviation angles is equal to or less than 25° is a product in which the compressing force F is exerted in the vertical direction with respect to the extrusion direction, and thus, it is possible to decrease the number of structural defects and to enhance the strength.

Fig. 14A and Fig. 14B show histograms showing the relationship between deviation angles of normals at the (0001) planes of metal crystals with respect to the direction parallel to the product surface and frequencies thereof for the material A and the material B, respectively.

Based on Figs. 14A and 14B, average values of the deviation angles and integrated widths for the respective compression methods are calculated and shown in Table 2.

{Table 2}

| MATERIAL | DEVIATION ANGLE FROM DIRECTION PARALLEL TO SURFACE | INTEGRATED WIDTH OF DEVIATION ANGLE |
|---|---|---|
| MATERIAL A | 81.2° | 17.0° |
| MATERIAL B | 81.7° | 53.0° |

According to Table 2, it is shown that, although the average values of the deviation angles are about 81° for both materials A and B, there is a large difference between the material A and the material B in terms of the integrated width. The integrated width is 17.0° for the material A, whereas the integrated width is 53° for the material B. Therefore, in the case in which, at a product surface, the average value of the deviation angles of the normals at the (0001) planes of metal crystals with respect to the direction parallel to the surface is equal to or greater than 80° and the integrated width of the deviation angles is equal to or less than 50°, the product can be identified as a product manufactured by the manufacturing method according to this embodiment.

(Example 2)

In Example 2, compression conditions are varied when exerting the compressing force F on the lump of magnesium-alloy material 2 (WE43) that is suitable for a medical application.

The lumps of magnesium-alloy material 2 having a diameter of 8 mm and a length of 12 mm were used as samples. The heating temperature in the compressing step S13 was changed so as to be 300 °C, 375 °C, and 450 °C; the reduction rate when compressing was changed so as to be 5 mm/s, 0.5 mm/s, and 0.05 mm/s; and results for these cases are shown in Table 3. Furthermore, the heating temperature was changed so as to be 350 °C, 400 °C, and 450 °C; the reduction rate when compressing was changed so as to be 1 mm/s, 0.1 mm/s, and 0.01 mm/s; and the results for these cases are shown in Table 4.

{Table 3}

| TEMPERATURE (°C) | REDUCTION RATE (mm/s) | SYMBOL | RESULT | HEIGHT OF COMPRESSED SAMPLE (mm) |
|---|---|---|---|---|
| 300 | 5 | - | CRACK | - |
| | 0.5 | - | CRACK | - |
| | 0.05 | - | CRACK | - |

(continued)

| TEMPERATURE (°C) | REDUCTION RATE (mm/s) | SYMBOL | RESULT | HEIGHT OF COMPRESSED SAMPLE (mm) |
|---|---|---|---|---|
| 375 | 5 | 375-5 | ✓ | 4.0 |
| | 0.5 | - | ✓ | 3.9 |
| | 0.05 | 375-005 | ✓ | 3.9 |
| 450 | 5 | 450-5 | ✓ | 3.8 |
| | 0.5 | - | ✓ | 3.8 |
| | 0.05 | 450-005 | ✓ | 3.7 |

{Table 4}

| TEMPERATURE (°C) | STRAIN RATE (1/s) | SYMBOL | COMPRESSION RESULT ("✓" means NO CRACK) | HEIGHT OF COMPRESSED SAMPLE (mm) | REDUCTION RATIO (%) |
|---|---|---|---|---|---|
| 350 | 1 | 350-1 | ✓ | - | 69 |
| | 0.1 | - | ✓ | - | 69 |
| | 0.01 | 350-001 | ✓ | - | 69 |
| 400 | 1 | - | ✓ | 4.0 | 69 |
| | 0.1 | - | ✓ | 3.9 | 68 |
| | 0.01 | - | ✓ | 3.9 | 70 |
| 450 | 1 | - | ✓ | 3.8 | 69 |
| | 0.1 | - | ✓ | 3.8 | 70 |
| | 0.01 | - | ✓ | 3.7 | 69 |

Based on Tables 3 and 4, it is preferable that the heating temperature when compressing the lump of magnesium-alloy material 2 be greater than 300 °C, and it is more preferable that the heating temperature be equal to or greater than 350 °C.

In addition, the relationship between true strain rate ε and load σ is separately shown for the case in which the heating temperature is 375 °C and the reduction rate is 0.05 mm in Fig. 15, for the case in which the heating temperature is 375 °C and the reduction rate is 5 mm/s in Fig. 16, for the case in which the heating temperature is 450 °C and the reduction rate is 0.05 mm/s in Fig. 17, for the case in which the heating temperature is 450 °C and the reduction rate is 5 mm/s in Fig. 18, for the case in which the heating temperature is 350 °C and the reduction rate is 1 mm/s in Fig. 19, and for the case in which the heating temperature is 350 °C and the reduction rate is 0.01 mm/s in Fig. 20.

Here, the reduction ratio can be expressed as:

```
Reduction ratio = ([thickness before compression] – [thickness

after compression])/[thickness before compression] × 100 (%).
```

According to Figs. 15 to 20, re-crystalized grain sizes reach near equilibrium at a true strain rate ε of about 0.6 when the compressing force F is gradually exerted on the lump of magnesium-alloy material 2. When calculating the reduction ratio based on this, the reduction ratio is determined to be about 45 %. Because the re-crystalized grain sizes reach near equilibrium at a reduction ratio of about 45 %, by compressing at a reduction ratio equal to or greater than 45 %, it is possible to enhance the corrosion resistance by making the material grain sizes uniform. Although WE43 is considered in this example, magnesium alloys other than WE43 may be considered.

(Example 3)

A case in which a shearing force is exerted at varying reduction rates in the state in which a plate-like magnesium-alloy material (WE43) having a thickness of 1 mm is heated to 350 °C will be described with reference to Figs. 21 to 22C.

Fig. 21 shows the relationship between the fraction of fractured surface portions (fraction of fractured surface) at the cut surface formed in the thickness direction and the reduction rate in the punching step S14. In addition, Figs. 22A to Fig. 22C are photomicrographs of the cut surface when the reduction rate is varied, respectively showing a case in which the reduction rate is 0.24 mm/s (Fig. 22A), a case in which the reduction rate is 1.44 mm/s (Fig. 22B), and a case in which the reduction rate is 1.92 mm/s (Fig. 22C). According to these figures, the fraction of fractured surface increases with an increase in the reduction rate, indicating that there is a substantially proportional relationship between the fraction of fractured surface and the reduction rate.

Therefore, by setting the reduction rate to be equal to or less than 1.5 mm/s based on the relationship shown in Fig. 21, it is possible to suppress the fractured surface portions at the cut surface in the plate-thickness direction to 50% or less. As a result, there is an advantage in that it is possible to enhance the strength of a product by decreasing of the factors causing stress concentration by decreasing the fraction of the fractured surface portions. Although WE43 is considered in this example, magnesium alloys other than WE43 may be considered.

{Reference Signs List}

S1 molding step
S2 grain-size adjusting step
S3 washing step
S4 checking step
F compressing force
1 extruded material (molded magnesium-alloy material)
2 lump of magnesium-alloy material
S11 extruding step
S12 cutting step
S13 compressing step
S14 punching step (shearing step)

**Claims**

1. An implant manufacturing method comprising:

   a molding step of molding a molded item by treating a raw-material piece constituted of a biodegradable metal material with plastic processing; and
   a grain-size adjusting step of increasing a metal grain size by applying a heat treatment to the molded item molded in the molding step.

2. The implant manufacturing method according to Claim 1, wherein the molding step includes:

   an extruding step of obtaining a plastically-deformed molded magnesium-alloy material by extruding a magnesium alloy;
   a cutting step of cutting the molded magnesium-alloy material obtained in the extruding step at an angle of 70° to 110° with respect to the extrusion direction; and
   a compressing step of exerting a compressing force on a lump of magnesium-alloy material obtained in the cutting step in a direction orthogonal to the extrusion direction.

3. The implant manufacturing method according to Claim 2, further comprising, before the compressing step or in the compressing step:

   a heating step of heating the lump of magnesium-alloy material.

4. The implant manufacturing method according to Claim 3, wherein, in the heating step, the lump of magnesium-alloy material is heated at a temperature that is greater than 300 °C and that is equal to or less than the melting point of the magnesium alloy.

5. The implant manufacturing method according to Claim 3, wherein, in the heating step, the lump of magnesium-alloy material is heated at a temperature that is equal to or greater than 350 °C and that is equal to or less than the melting point of the magnesium alloy.

6. The implant manufacturing method according to any one of Claims 3 to 5, wherein, in the compressing step, the lump of magnesium-alloy material is compressed at a reduction ratio equal to or greater than 45 %.

7. The implant manufacturing method according to any one of Claims 2 to 6, wherein, in the compressing step, the compressing force is exerted by using a metal mold in a state in which a lubricant is applied between the lump of magnesium-alloy material and the metal mold.

8. The implant manufacturing method according to Claim 7, wherein, in the compressing step, applying the lubricant between the metal mold and the lump of magnesium-alloy material and exerting the compressing force are repeated at least twice.

9. The implant manufacturing method according to any one of Claims 2 to 8, further comprising:

   a shearing step of cutting out a product from the compressed magnesium-alloy material after the compressing step,
   wherein the shearing step is performed at a reduction rate of 1.5 mm/s or less.

10. The implant manufacturing method according to Claim 1 or 2, further comprising:

    a washing step of washing a surface of the molded item molded in the molding step; and
    a checking step of checking the impurity concentration at the surface of the molded item that has been washed in the washing step,

    wherein, in the grain-size adjusting step, the heat treatment is applied to the molded item in the case in which the impurity concentration checked in the checking step is equal to or less than a predetermined value.

11. The implant manufacturing method according to Claim 10, wherein the washing step involves a treatment for peeling the surface of the molded item.

12. The implant manufacturing method according to Claim 11, wherein the washing step involves a treatment for dissolving the surface of the molded item by using an acid.

13. The implant manufacturing method according to Claim 11, wherein the washing step includes a treatment for dissolving the surface of the molded item by using an acid and a subsequent treatment for immersing the molded item in an alkaline solution.

14. The implant manufacturing method according to any one of Claims 10 to 13, wherein the grain-size adjusting step involves a solutionizing treatment.

15. The implant manufacturing method according to any one of Claims 10 to 13, wherein, in the grain-size adjusting step, an aging precipitation treatment is performed after a solutionizing treatment.

16. A magnesium-alloy implant in which c-axes of metal crystals are oriented in a main load direction.

17. The magnesium-alloy implant according to Claim 16, wherein an average value of deviation angles of normals at (0001) planes of metal crystals with respect to a thickness direction is equal to or less than 25°.

18. The magnesium-alloy implant according to Claim 16, wherein an average value of deviation angles of normals at (0001) planes of metal crystals with respect to a direction parallel to a surface is equal to or greater than 80°, and a width of a cumulative distribution of the deviation angles that are from 16 to 84 % of a maximum value of the deviation angles is equal to or less than 50°.

19. The magnesium-alloy implant according to any one of Claims 16 to 18 that is manufactured by punching processing.

20. The magnesium-alloy implant according to Claim 18, wherein a fraction of sheared portions at a cut surface along a thickness direction formed by punching processing is equal to or greater than 50 % relative to the thickness.

21. The magnesium-alloy implant according to any one of Claims 16 to 20, wherein, at a surface thereof, an Fe-ion concentration is equal to or less than 0.02 %, a Cu-ion concentration is equal to or less than 0.15 %, and a nickel concentration is equal to or less than 0.01 %.

FIG. 1

FIG. 2A

FIG. 2B

FIG. 3A

FIG. 3B

FIG. 4

|  | RAW-MATERIAL PIECE | AFTER STAMPING | AFTER HEAT TREATMENT |
|---|---|---|---|
| GRAIN SIZE | 50~60 $\mu$m | 20~30 $\mu$m | 50~60 $\mu$m |

FIG. 5

```
        START
          │
          ▼
      MOLDING ──── S1
          │
  ┌ ─ ─ ─ ┼ ─ ─ ─ ─ ─ ─ ─ ┐ ──── S2
  │       ▼                │
  │   SOLUTIONIZING        │
S21 ─┤  TREATMENT          │
  │       │                │
  │       ▼                │
  │  AGING PRECIPITATION   │
S22 ─┤   TREATMENT         │
  └ ─ ─ ─ ┼ ─ ─ ─ ─ ─ ─ ─ ┘
          │
          ▼
        END
```

FIG. 6A

FIG. 6B

## FIG. 7

| | RAW-MATERIAL PIECE | SOLUTIONIZING TREATMENT | SOLUTIONIZING TREATMENT + AGING PRECIPITATION TREATMENT |
|---|---|---|---|
| VICKERS HARDNESS | 69 | 79 | 85 |

## FIG. 8

START

MOLDING — S1

WASHING — S3

CHECKING — S4

CONCENTRATION ≦ THRESHOLD ? — S5

NO

YES

ADJUST GRAIN SIZE — S2

END

FIG. 9

```
        START

┌ ─ ─ ─ ─ ─ ─ ─ ─ ─ ─ ─ ─ ┐
      EXTRUDING STEP          S11
S1
        CUTTING STEP          S12

      COMPRESSING STEP        S13

       PUNCHING STEP          S14
└ ─ ─ ─ ─ ─ ─ ─ ─ ─ ─ ─ ─ ┘

S2    ADJUST GRAIN SIZE

         END
```

FIG. 10

FIG. 11

F

2

FIG. 12

F

C

(0001)

## FIG. 13A

MATERIAL A

FREQUENCY

0°  10°  20°  30°  40°  50°  60°  70°  80°  90°

(0001) DEVIATION ANGLE OF NORMAL OF PLANE
FROM PLATE-THICKNESS DIRECTION

## FIG. 13B

MATERIAL B

FREQUENCY

0°  10°  20°  30°  40°  50°  60°  70°  80°  90°

(0001) DEVIATION ANGLE OF NORMAL OF PLANE
FROM PLATE-THICKNESS DIRECTION

# FIG. 14A

MATERIAL A

(0001) DEVIATION ANGLE OF NORMAL OF PLANE
FROM DIRECTION PARALLEL TO SURFACE

# FIG. 14B

MATERIAL B

(0001) DEVIATION ANGLE OF NORMAL OF PLANE
FROM DIRECTION PARALLEL TO SURFACE

# FIG. 15

# FIG. 16

## FIG. 17

## FIG. 18

# FIG. 19

# FIG. 20

## FIG. 21

## FIG. 22A

## FIG. 22B

1. 44mm/s

50%

## FIG. 22C

1. 92mm/s

80%

## INTERNATIONAL SEARCH REPORT

| International application No. |
| --- |
| PCT/JP2013/075635 |

A.  CLASSIFICATION OF SUBJECT MATTER

*C22F1/06*(2006.01)i, *A61F2/28*(2006.01)i, *A61L27/00*(2006.01)i, *B21C23/00* (2006.01)i, *B21C23/08*(2006.01)i, *B21J5/00*(2006.01)i, *B21K1/76*(2006.01)i, *C23G1/12*(2006.01)i, *C22C23/06*(2006.01)n, *C22F1/00*(2006.01)n

According to International Patent Classification (IPC) or to both national classification and IPC

B.  FIELDS SEARCHED

Minimum documentation searched (classification system followed by classification symbols)

C22F1/00-3/02, C22C1/00-49/14, A61F2/28, A61L27/00, B21C23/00, B21C23/08, B21J5/00, B21K1/76, C23G1/12

Documentation searched other than minimum documentation to the extent that such documents are included in the fields searched

| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
| --- | --- | --- |
| X<br>A | JP 2009-535504 A  (Biomagnesium Systems Ltd.),<br>01 October 2009 (01.10.2009),<br>claims; paragraph [0280]; example 1; paragraphs [0309], [0311]<br>& US 2009/0081313 A1     & EP 2021522 A<br>& WO 2007/125532 A2     & CA 2645737 A<br>& KR 10-2008-0113280 A  & CN 101484599 A<br>& IL 194910 D            & AU 2007245256 A<br>& MX 2008013652 A        & BRA PI0710355 | 1<br>2-21 |
| A | WO 2007/058276 A1  (Independent Administrative Institution National Institute for Materials Science),<br>24 May 2007 (24.05.2007),<br>& US 2009/0171452 A1     & EP 1959025 A1<br>& WO 2007/058276 A1 | 1-21 |

☒  Further documents are listed in the continuation of Box C.  ☐  See patent family annex.

| \* | Special categories of cited documents: |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>    20 November, 2013 (20.11.13) | Date of mailing of the international search report<br>    03 December, 2013 (03.12.13) |
| --- | --- |
| Name and mailing address of the ISA/<br>    Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2013/075635

| C (Continuation). DOCUMENTS CONSIDERED TO BE RELEVANT | | |
|---|---|---|
| Category* | Citation of document, with indication, where appropriate, of the relevant passages | Relevant to claim No. |
| A | JP 2013-524004 A (Magnesium Electron Ltd.), 17 June 2013 (17.06.2013), & US 2013/0195714 A & GB 201005031 D & GB 201005031 D0 & EP 2550377 A & WO 2011/117628 A & CN 102892909 A | 1-21 |

Form PCT/ISA/210 (continuation of second sheet) (July 2009)

## INTERNATIONAL SEARCH REPORT

| | International application No. |
|---|---|
| | PCT/JP2013/075635 |

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

    The invention according to claim 1 is an invention relating to a method
for producing an implant, said method comprising a specific "shaping step"
and a specific "particle size-controlling step".  The inventions according
to claims 2 to 15 are inventions relating to a method for producing an implant
wherein further technical regulations are added to the invention according
to claim 1.
(Continued to extra sheet)

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2013/075635

Continuation of Box No.III of continuation of first sheet(2)

However, the invention according to claim 16 is an invention relating to an implant wherein "the axis (c) of metal crystals is oriented along the main loading direction". Thus, it cannot be considered that there are the same or corresponding special technical features among the inventions respectively according to claims 1 to 15.

Further, the above-said opinion may be also applied to claims 17-21.

Form PCT/ISA/210 (extra sheet) (July 2009)

**EP 3 050 996 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009178293 A

- JP 4150219 B